# EUROPÄISCHE PATENTSCHRIFT

(11) **EP 4 279 208 B1**
(45) Veröffentlichungstag und Bekanntmachung des Hinweises auf die Patenterteilung: **15.07.2026**
(21) Anmeldenummer: 23000070.5
(22) Anmeldetag: 05.05.2023
(51) Int. Cl.: B23K 9/095, B23K 9/32, G01R 19/00, A61F 9/06

(54) **STROMSTÄRKEANZEIGER FÜR SCHWEISSHELM**
WELD HELMET CURRENT MONITORING
INDICATEUR DE COURANT POUR CASQUE DE SOUDAGE

(30) Priorität: 18.05.2022 DE 102022001631
(43) Veröffentlichungstag der Anmeldung: 22.11.2023
(73) Patentinhaber: Meyer, Christian, 24894 Tolk (DE)
(72) Erfinder: Meyer, Christian, 24860 Klappholz (DE)

(56) Entgegenhaltungen:
- WO-A2-2016/016445
- CN-U- 205 790 766
- CN-U- 211 554 112
- US-A- 4 638 146
- US-A1- 2010 090 683
- US-A1- 2010 237 853
- US-A1- 2017 021 444
- US-A1- 2018 120 362
- US-A1- 2018 207 741
- US-A1- 2021 161 717
- US-B2- 10 660 796
- ANONYMOUS: "Wireless Battery-Powered Hall Effect Proximity Sensor | iQunet.com", 23 September 2021 (2021-09-23), XP093293115, Retrieved from the Internet <URL:https://web.archive.org/web/20210923100201/https://iqunet.com/shop/all-products/sensors/wireless-battery-powered-hall-effect-proximity-sensor/#expand>
- LENZ MARTIN ET AL: "On Using Off-the-Shelf Micro Projectors for 3D Metrology", 15 May 2009 (2009-05-15), OAGM/AAPR Workshop 2009, Stainz, Austria, XP093293702, Retrieved from the Internet <URL:https://oagm09.aapr.at/papers/p41.pdf>

## Beschreibung

Zur Qualitätssicherung beim Ausführen einer Schweißnaht ist die Überwachung der momentanen Stromstärke während des Schweißens von überragender Bedeutung, da dies Auskunft über den Fluss der Schweißelektrode, das Schweißbad und die Verbindung mit dem Grundwerkstoff gibt. Eine rissfreie, nicht verschlackte Schweißnaht, keine Mikrorisse in der Wärmeeinflusszone garantieren eine Schweißverbindung, welche dieselben mechanischen Eigenschaften hinsichtlich des Lastfalles aufweisen, wie der Grundwerkstoff.

### Stand der Technik

Es gibt viele Vorschläge, die während der Schweißausführung vorhandenen Parameter zu erfassen und diese unverzüglich anzuzeigen.

Die US 2017/021 444 A1 zeigt ein System umfassend einen Schweißhelm und einen Stromstärkeanzeiger, wobei der Stromstärkeanzeiger einen Stromsensor aufweist, wobei der Stromsensor als Hall-Sensor ausgebildet ist und dazu eingerichtet ist, ein Stromkabel eines Schweißtransformators umzugreifen, einen mit dem Stromsensor verbundenen Sender, einen Empfänger, der im Schweißhelm befestigt ist (der Schweißhelm hat einen Empfänger).

Die US 4 638 146 A zeigt ein Verfahren zum Schutz der Augen vor Schweißstrahlen beim Lichtbogenschweißen und eine Vorrichtung dafür, die in der Lage sind, eine Filterplatte über einen Zeitraum von unmittelbar vor der Erzeugung von Lichtbogenschweißstrahlen bis zur Beendigung der Erzeugung zu schließen, um die Strahlen vollständig abzufangen, um dadurch die Augen eines Bedieners wirksam vor den Strahlen zu schützen. Die Erfindung ist so aufgebaut, dass eine Steuerung die Ein-Aus-Steuerung einer von einer Filterstromquelle an eine optisch transparente Filterplatte, bestehend aus elektrooptischem Keramikmaterial, angelegten Spannung in Abhängigkeit von einem von einer Erfassungsschaltung zugeführten Signal durchführt, wenn die Erfassungsschaltung einen in einem Schweißstrom auftretenden Aufbaustrom erfasst, um dadurch die Betätigung der Filterplatte auszuführen. Aufgabe dieser Erfindung ist es, ein Verfahren zum Schutz der Augen vor Schweißstrahlen beim Lichtbogenschweißen bereitzustellen.

Die US 2021/161717 A1 bezieht sich im Allgemeinen auf einen selbstverdunkelnden Schweißhelm und ein Schweißgerät, das mit dem selbstverdunkelnden Schweißhelm ausgestattet ist. Es wird ein verbesserter selbstverdunkelnder Schweißhelm vorzuschlagen, so dass seine Betriebsparameter automatisch in Abhängigkeit von den Betriebsparametern eines relevanten elektrischen Schweißgeräts vernünftig eingestellt werden können, bevor er von einem Bediener getragen wird. Anschließend kann der Bediener den Schweißhelm direkt auf dem Kopf tragen, um bequem einen Schweißvorgang durchzuführen. Die Schweißvorrichtung umfasst eine elektrische Schweißmaschine, eine Gasflasche (z.B. eine Argongasflasche) und einen Schweißbrenner. Die elektrische Schweißmaschine, die Gasflasche und der Schweißbrenner sind in an sich bekannter Weise durch Kabel oder Rohrleitungen miteinander verbunden. Außerdem umfasst die Schweißvorrichtung auch ein drahtloses Kommunikationsmodul, das beispielsweise an der elektrischen Schweißmaschine angeordnet ist. Das drahtlose Kommunikationsmodul ist mit einer inneren elektrischen Schaltung der elektrischen Schweißmaschine, um Betriebsparameter der elektrischen Schweißmaschine wie Schweißstrom, Schweißart, Schweißmaterialart, Lötdrahtgröße oder dergleichen und eine sofortige Meldung zu übertragen. Ein Schweißhelm ist mit einem drahtlosen Kommunikationsmodul versehen. In dieser Druckschrift ist über die drahtlose Kommunikation nichts weiter ausgeführt.

Die US 10 660 796 B2 Patent schützt einen drahtlosen, projektorartigen, selbstverdunkelnden Schweißheim, der Folgendes umfasst: ein Helmgehäuse; eine Kopfbandstruktur zum Befestigen des Helmgehäuses; einen selbstverdunkelnden Filter, der an dem Helmgehäuse befestigt ist; ein drahtloses Kommunikationsmodul und einen Mikroprojektor, der mit dem Selbstverdunkelungsfilter und dem drahtlosen Kommunikationsmodul datenverbunden ist, wobei der Mikroprojektor, wenn sich der Selbstverdunkelungsfilter in einem transparenten Zustand befindet, so angepasst werden kann, dass er Betriebsparameter des Selbstverdunkelungsfilters und/oder von dem drahtlosen Kommunikationsmodul empfangene Daten als Bilder auf eine Ebene vor dem Schweißhelm projiziert, so dass sie von einem Bediener, der den Schweißheim auf seinem Kopf trägt, beobachtet werden können. Die vorliegende Anwendung offenbart auch eine Schweißvorrichtung, die mit dem drahtlosen Schweißhelm mit automatischer Verdunkelung ausgestattet ist. Die drahtlosen Kommunikationsmodule können jeweils ein auf Wireless Fidelity basierendes Modul (WIFI-Module), ein Bluetooth-Modul, ein Infrarot-Datenmodul oder ein anderes geeignetes Kommunikationsmodul sein, das eine drahtlose Datenverbindung durchführen kann. Weitere Angaben zur Datenübertragung sind in dieser Patentschrift nicht enthalten.

Aus der EP 3 462 184 A1 eine Erfassungsvorrichtung für eine aktive Blendschutzvorrichtung mit Blendschutzfilter bekannt, bei der eine Erfassungseinheit zumindest einen Stromsensor aufweist, welcher zu einer Erfassung zumindest eines Schweißparameters des Schweißgeräts vorgesehen ist und variabel an ein Stromkabel des Schweißgeräts befestigt werden kann, und eine Kommunikationseinheit, welche zu einer Übertragung des zumindest einen Schweißparameters an die aktive Blendschutzvorrichtung, besonders Blendschutzfilter, vorgesehen ist. Blendschutzvorrichtung und der Stromsensor am Stromkabel des Schweißgeräts sind kabellos miteinander verbunden. Die Erfassungsvorrichtung ist vorzugsweise als eine Art Schelle ausgebildet, die aus zwei Hälften besteht und die werkzeuglos um das Stromkabel herummontiert und entfernt werden kann. Der Blendschutzfilter wird abhängig vom erfassten Schweißparameter abgedunkelt.

Es handelt sich somit um einen automatisierten Augenschutz für die die Schweißung vornehmende Person. Die Stromstärke wird der schweißenden Person im Blendschutzfilter nicht angezeigt.

### Offenbarung der Erfindung

Die mit der vorliegenden Erfindung zu lösende Aufgabe besteht somit darin, die gemessene momentane Stromstärke der schweißenden Person unabhängig von der Schweißausrüstung anzuzeigen.

Ausgehend von der US 2017/021 444 A1 wird dieses Problem mit den Merkmalen im Patentanspruch 1 und 2 gelöst. Weitere sinnvolle Weiterbildungen der Erfindung sind durch die Merkmale der Patentansprüche 3 bis 5 festgelegt.

System umfassend einen Schweißhelm und einen Stromstärkeanzeiger, der Stromstärkeanzeiger weist einen Stromsensor auf, wobei der Stromsensor als Hall-Sensor ausgebildet ist und dazu eingerichtet ist, ein Stromkabel eines Schweißtransformators zu umgreifen, einen mit dem Stromsensor verbundenen Sender, einen Empfänger, der im Schweißhelm befestigt ist, wobei der Sender als Client und der Empfänger als Server arbeiten, dass Client und Server mittels WLAN aufeinander abgestimmt unveränderbar fest funkverbunden sind und der Server eine Projektionseinrichtung zu einem Sichtschutz hin aufweist oder ein eigenes Display aufweist, wobei der Server dazu eingerichtet ist, die empfangenen Werte der Stromstärke mit der Projektionseinrichtung in den Sichtschutz zu projizieren oder im Display anzuzeigen.

Dadurch dass der Sender als Client und der Empfänger als Server arbeiten, dass Client und Server mittels WLAN aufeinander abgestimmt unveränderbar fest funkverbunden sind und der Server die empfangenen Werte der Stromstärke auf einen Sichtschutz des Schweißhelms oder einem eigenen Display des Servers projizieren, ist die schweißende Person zu jedem Zeitpunkt über die Stromstärke und somit über den Materialfluss in der Schmelze und die Größe des Schmelzbades informiert. Da Client und Server auf die gleiche festgelegte, unveränderbare WLAN-Frequenz abgestimmt sind, können sie gemeinsam ohne weitere Einstellungen verwendet werden. Dieses Paar Client Server kann an beliebigen Schweißtransformatoren und Schweißheimen bzw. Handschilden festgeklippt werden. Folglich ist die universelle Benutzung gesichert. Da die Messwerte der Stromstärke entweder in den Sichtschutz des Schweißhelmes projiziert oder vom einem Display angezeigt werden, sind keine weiteren Vorrichtungen erforderlich. Dies ermöglicht eine einfache Handhabung.

Da der Server nach Patentanspruch 1 oder 2 festgeklippt wird, findet er nicht nur einen Platz im Schweißhelm, sondern kann auch im Schweißerhandschild befestigt werden.

Die jeweils eigene Stromversorgung, nach Patentanspruch 4, von Client und Server in Form von Batterien oder Akkus macht weitere Stromanschlüsse unnötig und verbessert so die universelle Verwendung.

Das abgestimmte Übertragungsprotokoll nach Patentanspruch 5 zwischen Server und Client sichert die Kommunikation zwischen den einander zugeordneten Geräten, ohne das weitere Störungen durch andere sendende Geräte auftreten.

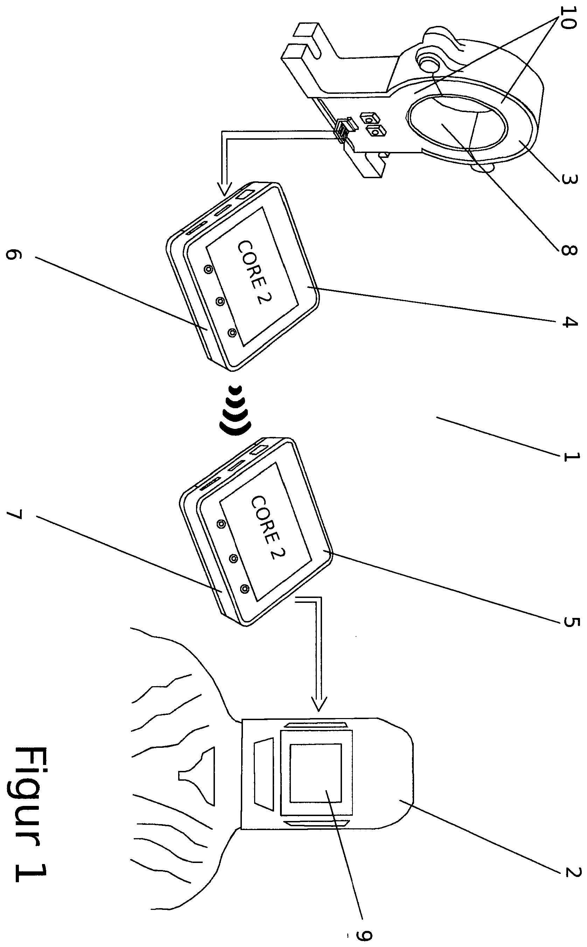

Die nachfolgende Beschreibung eines Ausführungsbeispiels der Erfindung dient im Zusammenhang mit der Figur 1 der näheren Erläuterung. Es zeigt:
- Figur 1: alle Teile des Stromsensors

Figur 1 zeigt einen Stromstärkeanzeiger 1 für einen Schweißheim 2 mit einem Stromsensor 3, der ein nicht dargestelltes Stromkabel eines ebenfalls nicht dargestellten Schweißtransformators umgreift. Dazu ist der Stromsensor 3 in zwei Hälften 10 aufgeteilt, die von Hand ohne Werkzeug miteinander verbunden und gelöst werden können, sodass die Hälften 10 das Stromkabel des Schweißtransformators fest umschließen. Im Stromsensor 3 ist zur Messung der Stromstärke ein Hall-Sensor 8 eingebaut. An dem Gehäuse des Stromsensors 3 ist der Sender 4, der die Funktion eines Client 6 hat, angesteckt. Dazu verfügt der Stromsensor 3 über eine Ansteckzunge mit Leiterbahnen, die in eine entsprechende Buchse im Sender 4 eingreift. Der Sender 4 kann auf den Stromsensor 3 aufgesteckt werden und verfügt über eine eigene, eingebaute Stromversorgung, wobei diese eine Batterie oder ein Akku sein kann. Diese Stromversorgung versorgt auch den Hall-Sensor 8 im Stromsensor 3. Ein Empfänger 5, der grundsätzlich baugleich wie der Sender 4 ist, kann in einem Schweißhelm 2 mittels Klipps angebracht werden. Alternativ kann der Empfänger 5 an einem Handschild mittels Klipps angebracht werden. Der Empfänger 5 übernimmt die Funktion eines Servers 7. Die empfangenen Daten werden durch eine Projektionseinrichtung auf den Sichtschutz 9 geworfen, damit die schweißende Person fortlaufend über die augenblickliche Stromstärke informiert ist. Wie der Sender 4 verfügt der Empfänger 5 über eine eigene Stromversorgung.

Server 7 und Client 6 kommunizieren über WLAN. Das Übertragungsprotokoll ist so auf Server 7 und Client 6 abgestimmt, dass nur diese beiden untereinander Daten austauschen können. Dazu dient eine Verschlüsselung, deren Schlüssel einmalig auf dem Paar aus Server 7 und Client 6 installiert ist.

Möchte eine Person eine Schweißnaht kontrolliert herstellen, befestigt sie den Stromsensor 3 mit dem Sender 4 am Stromkabel und klippt dem Empfänger 5 beispielsweise im Schweißhelm 2 fest. Danach kann die Person die Schweißung beginnen und wird dabei laufend über die Stromstärke informiert. Da Server 7 und Client 6 fest aufeinander abgestimmt sind, können beide Geräte von verschiedenen Personen und/oder an verschiedenen Arbeitsplätzen verwendet werden.

Der Empfänger verfügt entweder über eine Projektionseinrichtung, welche die Werte der Stromstärke im Sichtfeld der schweißenden Person auf den Sichtschutz 9 wirft oder über ein Display, welches am Empfänger befestigt ist und von der schweißenden Person abgelesen wird.

### Bezugszeichenliste

- 1: Stromstärkeanzeiger
- 2: Schweißhelm
- 3: Stromsensor
- 4: Sender
- 5: Empfänger
- 6: Client
- 7: Server
- 8: Hall-Sensor
- 9: Sichtschutz
- 10: Hälfte

Es folgt eine Seite mit Zeichnung.

## Patentansprüche

1. System umfassend einen Schweißhelm (2) und einen Stromstärkeanzeiger (1), der Stromstärkeanzeiger (1) aufweisend einen Stromsensor (3), wobei der Stromsensor (3) als Hall-Sensor (8) ausgebildet ist und dazu eingerichtet ist, ein Stromkabel eines Schweißtransformators umzugreifen, einen mit dem Stromsensor (3) verbundenen Sender (4), einen Empfänger (5), der im Schweißheim (2) befestigt ist, **dadurch gekennzeichnet, dass** der Sender (4) als Client (6) und der Empfänger (5) als Server (7) arbeiten, dass Client (6) und Server (7) mittels WLAN aufeinander abgestimmt unveränderbar fest funkverbunden sind und der Server (7) eine Projektionseinrichtung zu einem Sichtschutz (9) hin aufweist oder ein eigenes Display aufweist, wobei der Server (7) dazu eingerichtet ist, die empfangenen Werte der Stromstärke mit der Projektionseinrichtung in den Sichtschutz (7) zu projizieren oder im Display anzuzeigen.

2. System umfassend ein Schweißerhandschild und einen Stromstärkeanzeiger (1), der Stromstärkeanzeiger (1) aufweisend einen Stromsensor (3), wobei der Stromsensor (3) als Hall-Sensor (8) ausgebildet ist und dazu eingerichtet ist, ein Stromkabel eines Schweißtransformators umzugreifen, einen mit dem Stromsensor (3) verbundenen Sender (4), einen Empfänger (5), der im Schweißerhandschild befestigt ist, **dadurch gekennzeichnet, dass** der Sender (4) als Client (6) und der Empfänger (5) als Server (7) arbeiten, dass Client (6) und Server (7) mittels WLAN aufeinander abgestimmt unveränderbar fest funkverbunden sind und der Server (7) eine Projektionseinrichtung zu einem Sichtschutz (9) hin aufweist oder ein eigenes Display aufweist, wobei der Server (7) dazu eingerichtet ist, die empfangenen Werte der Stromstärke mit der Projektionseinrichtung in den Sichtschutz (7) zu projizieren oder im Display anzuzeigen.

3. System nach Patentanspruch 1 oder Patentanspruch 2, **dadurch gekennzeichnet, dass** der Server (7) im Schweißhelm (2) oder dem Schweißerhandschild mittels Klipps befestigbar ist.

4. System nach einem der vorhergehenden Patentansprüche, **dadurch gekennzeichnet, dass** Server (7) und Client (6) über eine jeweils eigene Stromversorgung verfügen, bestehend aus Batterie oder Akku.

5. System nach einem der vorhergehenden Patentansprüche, **dadurch gekennzeichnet, dass** Server (7) und Client (8) mit einem Übertragungsprotokoll kommunizieren, welches auf sie abgestimmt ist.

## Claims

1. A system comprising a welding helmet (2) and a current indicator (1), the current indicator (1) comprising a current sensor (3), wherein the current sensor (3) is designed as a Hall sensor (8) and is configured to encircle a power cable of a welding transformer, a transmitter (4) connected to the current sensor (3), a receiver (5) mounted in the welding helmet (2), **characterized in that** the transmitter (4) operates as a client (6) and the receiver (5) as a server (7), that the client (6) and the server (7) are permanently and unalterably connected to each other via a coordinated wireless connection, and the server (7) includes a projection device directed toward a visor (9) or has its own display, wherein the server (7) is configured to project the received current values onto the visor (9) using the projection device or to display them on the display.

2. A system comprising a welder's hand shield and a current indicator (1), the current indicator (1) comprising a current sensor (3), wherein the current sensor (3) is designed as a Hall sensor (8) and is configured to encircle a power cable of a welding transformer, a transmitter (4) connected to the current sensor (3), a receiver (5) mounted in the welder's hand shield, **characterized in that** the transmitter (4) functions as a client (6) and the receiver (5) as a server (7), that the client (6) and server (7) are permanently and unalterably connected to each other via a coordinated wireless connection, and the server (7) includes a projection device directed toward a privacy screen (9) or has its own display, wherein the server (7) is configured to project the received current values onto the privacy screen (9) using the projection device or to display them on the display.

3. A system according to claim 1 or claim 2, **characterized in that** the server (7) can be attached to the welding helmet (2) or the welder's hand shield by means of clips.

4. A system according to any of the preceding claims, **characterized in that** the server (7) and the client (6) each have their own power supply, consisting of a battery or rechargeable battery.

5. A system according to any of the preceding patent claims, **characterized in that** the server (7) and the client (8) communicate using a transmission protocol tailored to them.

## Revendications

1. Système comprenant un casque de soudage (2) et un indicateur d'intensité de courant (1), l'indicateur d'intensité de courant (1) comportant un capteur de courant (3), le capteur de courant (3) étant conçu comme un capteur à effet Hall (8) et étant agencé pour entourer un câble d'alimentation d'un transformateur de soudage, un émetteur (4) relié au capteur de courant (3), un récepteur (5) fixé dans le casque de soudage (2), **caractérisé en ce que** l'émetteur (4) fonctionne comme client (6) et le récepteur (5) comme serveur (7), **en ce que** le client (6) et le serveur (7) sont reliés entre eux de manière fixe et immuable par une liaison radio via Wi-Fi et que le serveur (7) comporte un dispositif de projection vers un écran de protection (9) ou dispose de son propre écran, le serveur (7) étant conçu pour projeter les valeurs de courant reçues sur l'écran de protection (9) à l'aide du dispositif de projection ou pour les afficher sur son écran.

2. Système comprenant un panneau de signalisation pour soudeur et un indicateur d'intensité de courant (1), l'indicateur d'intensité de courant (1) comportant un capteur de courant (3), le capteur de courant (3) étant réalisé sous la forme d'un capteur à effet Hall (8) et étant agencé pour entourer un câble d'alimentation d'un transformateur de soudage, un émetteur (4) relié au capteur de courant (3), un récepteur (5) fixé dans le bouclier de soudeur, **caractérisé en ce que** l'émetteur (4) fonctionne comme client (6) et le récepteur (5) comme serveur (7), **en ce que** le client (6) et le serveur (7) sont reliés entre eux de manière fixe et immuable par une liaison radio via WLAN et que le serveur (7) comporte un dispositif de projection vers un écran de protection (9) ou dispose de son propre écran, le serveur (7) étant conçu pour projeter les valeurs de courant reçues sur l'écran de protection (9) à l'aide du dispositif de projection ou pour les afficher sur son écran.

3. Système selon la revendication 1 ou la revendication 2, **caractérisé en ce que** le serveur (7) peut être fixé dans le casque de soudage (2) ou sur le bouclier de soudage à l'aide de clips.

4. Système selon l'une des revendications précédentes, **caractérisé en ce que** le serveur (7) et le client (6) disposent chacun de leur propre alimentation électrique, composée d'une pile ou d'une batterie rechargeable.

5. Système selon l'une des revendications précédentes, **caractérisé en ce que** le serveur (7) et le client (8) communiquent à l'aide d'un protocole de transmission qui leur est adapté.
